# DEMANDE DE BREVET EUROPEEN

(11) **EP 0 882 980 A1**
(43) Date de publication de la demande: **09.12.1998**
(21) Numéro de dépôt: 98401345.8
(22) Date de dépôt: 04.06.1998
(51) Int. Cl.: G01N 27/327, G01N 33/543

(54) **Traitement de surface d'un substrat limitant sa fluorescence naturelle**

(30) Priorité: 06.06.1997 FR 9707046
(71) Demandeur: COMMISSARIAT A L'ENERGIE ATOMIQUE, 75015 Paris (FR)
(72) Inventeur: Caillat, Patrice, 38130 Echirolles (FR); Livache, Thierry, 38560 Jarrie (FR)
(74) Mandataire: Dubois-Chabert, Guy

(57) **Abrégé**

L'invention concerne un support d'électrodes, ce support comprenant un substrat (22, 72) et des électrodes (24, 74) qui sont formées sur ce substrat, ce support étant tel qu'au moins une partie d'une zone entourant au moins une électrode est non fluorescente à une certaine longueur d'onde, dite longueur d'onde de fluorescence, lorsque cette partie est soumise à une lumière ayant une autre longueur d'onde, dite longueur d'onde d'excitation.

## Description

### Domaine technique

La présente invention concerne un support d'électrodes comportant au moins une électrode pouvant être recouverte par un dépôt électrochimique, ainsi qu'éventuellement, un système de lecture de ce support.

La présente invention concerne également un substrat formant support pour des réactifs, ce substrat incorporant, ou pas, des liaisons électriques.

L'invention s'applique notamment à la fabrication de capteurs ou d'autres éléments sensibles miniaturisés dans lesquels des puces munies d'un grand nombre d'électrodes ou de sites doivent être réalisées.

Ces électrodes ou ces sites, pour être adaptées à leurs fonctions spécifiques dans le capteur ou l'élément sensible, doivent être recouvertes de matériaux appropriés.

A titre d'exemple, l'invention s'applique à la réalisation d'éléments miniaturisés tels que des "biochips" qui sont des puces qui peuvent ou non comporter une partie de circuit électrique réalisée sur un substrat, tel qu'un champ d'électrodes, et une partie biologique réalisée à la surface de la puce.

Dans cet exemple, il convient de déposer sélectivement sur les électrodes ou les sites des composés chimiques compatibles avec les produits biologiques.

D'une manière générale, la micro-électronique classique est de plus en plus appelée à être un maillon de système beaucoup plus complexes dans lesquels plusieurs fonctions sont intégrées. Ces systèmes, ou microsystèmes, peuvent être intégrés dans des applications allant des capteurs physiques aux derniers développement de puces dites "biologiques".

Dans certains cas une cellule sensible capable de mesurer un phénomène physique est associée à un circuit intégré capable d'assurer le traitement de l'information et son exploitation (ex : Air-Bag pour automobile).

Dans d'autres cas, un circuit intégré va subir une finition lui permettant d'être utilisé dans un milieu biologique (ex : mesureur de glucose intégré, sondes de pression sanguine).

### Etat de la technique antérieure

Dans les systèmes d'analyse biologique, on met en contact une zone, ou un réactif, avec une solution à analyser.

La réactivité obtenue est mesurée, par exemple, par une des méthodes suivantes :
- mesure électrique par impédance-métrie,
- microbalance,
- mesure optique par changement d'indice de réfraction,
- marquage radioactif,
- fluorescence.

Cette dernière méthode (fluorescence) est de plus en plus utilisée car relativement facile à mettre en oeuvre et de bonne sensibilité.

Schématiquement, elle consiste à placer dans l'analyte à tester (liquide, la plupart du temps) un fluophore. L'analyte est mise en contact avec le réactif fixé localement sur le microsystème. Si il y a réaction-appariement de quelque nature qu'elle soit il reste, sur la zone de test, une partie du réactif associé à une partie de l'analyte portant le fluophore. Après lavage, une lecture de la fluorescence permet de déterminer, si oui ou non, il y a eu appariement sur le site porteur.

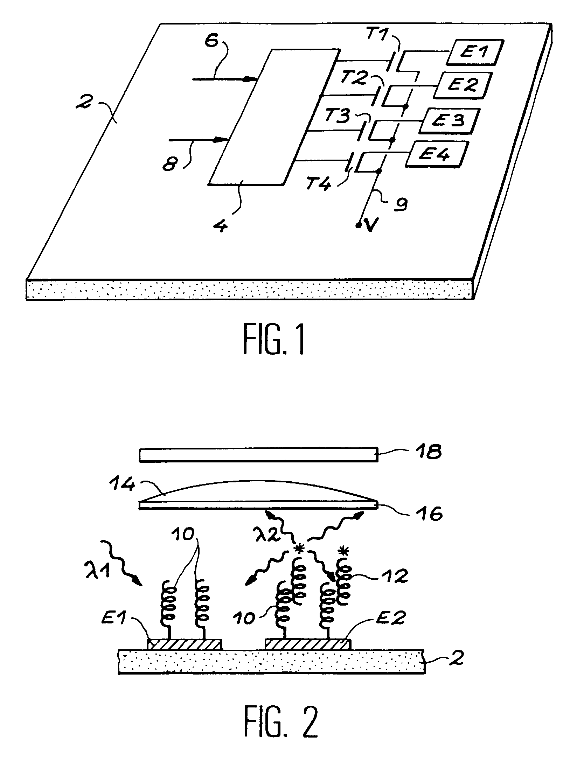

Un exemple d'un tel système va être décrit plus précisément en liaison avec les figures 1 à 3, dans le cas de la reconnaissance anticorps-antigène ou sonde ADN-ADN d'un patient.

On voit sur la figure 1 un substrat 2 par exemple en silicium.

La surface de ce substrat 2 est rendue fonctionnelle par greffage de molécules-sondes.

De plus, cette surface est rendue sélectivement fonctionnelle par adressage sélectif d'une électrode choisie parmi une pluralité d'électrodes indépendantes E1, E2, E3 et E4, par exemple l'électrode E2, les autres électrodes n'étant pas sélectionnées.

L'adressage d'une électrode correspond à l'application à celle-ci d'une tension différente de la tension de référence d'un bain non représenté, dans lequel les molécules-sondes sont diluées.

Cette tension, différente de la tension de référence, est suffisamment élevée pour provoquer soit un phénomène d'électrodéposition (dans le cas d'une utilisation de polymères conducteurs du type polypyrrol ou polyaniline qui sont porteurs des molécules-sondes), soit un transfert de charges irréversible (dans le cas d'une réaction de greffage covalent).

Cet adressage est obtenu en réalisant un circuit de commande 4 sur la surface du substrat 2 où sont les électrodes.

Dans l'exemple de la figure 1, ce circuit de commande 4 est un multiplexeur qui reçoit des données de poids fort et des données de poids faible par l'intermédiaire de lignes électriques 6 et 8 et qui commande les quatre électrodes indépendantes E1, E2, E3 et E4, respectivement par l'intermédiaire de quatre transistors T1, T2, T3 et T4.

On voit également sur la figure 1 une ligne 9 qui permet d'appliquer une tension V aux sources des transistors de sortie du multiplexeur 4.

Cette tension V sert à polariser les électrodes sélectionnées grâce à ce multiplexeur 4.

La lecture des réactions sur les électrodes, ou plus exactement de ce qui s'est apparié sur celles-ci, s'effectue par voie optique, par exemple en utilisant un système optique comme l'illustre schématiquement la figure 2.

Sur la figure 2, on voit le substrat 2 en coupe transversale schématique et partielle ainsi que seulement deux des électrodes E1 à E4, par exemple l'électrode E1 et l'électrode E2.

Ces électrodes E1 à E4 portent toutes des molécules-sondes 10.

Cependant certaines de ces molécules-sondes, par exemple celles de l'électrode E2, sont susceptibles d'être hybridées à des branches d'ADN 12 tandis que les autres ne le sont pas.

Ces branches d'ADN 12 sont marquées par un produit de marquage flucrescent de telle manière que, lorsque les branches d'ADN ainsi marquées sont éclairées au moyen d'une lumière de longueur d'onde λ₁ provenant d'une source lumineuse non représentée, ces branches marquées émettent une lumière ayant une autre longueur d'onde λ₂.

Une lentille 14, qui est munie d'un filtre optique 16 capable d'arrêter la lumière de longueur d'onde λ₁ et de laisser passer la lumière de longueur d'onde λ₂ est disposée au-dessus de la surface du substrat 2, en regard des électrodes.

Des moyens de détection 18, qui comprennent par exemple des dispositifs à couplage de charges, sont disposés au-dessus de la lentille 14, cette lentille 14 étant ainsi comprise entre les moyens de détection 18 et la surface du substrat 2.

Ces moyens de détection 18 permettent de détecter la lumière de longueur d'onde λ₂ qui est focalisée sur les dispositifs à couplage de charges.

### Exposé de l'invention

Dans tous les cas, exposés ci-dessus, et plus particulièrement lorsque le substrat support est complexe et est réalisé par les techniques de micro-électronique, il existe une limitation à la sensibilité de la mesure.

Cette limitation est due à la fluorescence intrinsèque du substrat, qui génère un bruit de fond lors de la mesure après mise en contact du réactif et de l'analyte marqué par fluorophore.

Par exemple, dans une technologie standard de micro-électronique, il est couramment fait appel, pour la fabrication des transistors, à des couches de passivation dopées au phosphore. Ces couches ont immanquablement, lors de l'illumination finale, une fluorescence intrinsèque qui gêne fortement l'analyse du résultat.

Afin d'éviter de tels inconvénients, l'invention a pour objet un support d'électrodes, ce support comprenant un substrat et des électrodes qui sont formées sur ce substrat, ce support étant tel qu'au moins une partie d'une zone, ou une zone, entourant au moins une électrode est non fluorescente à au moins une certaine longueur d'onde (longueur d'onde de fluorescence) lorsque cette partie est soumise à une lumière ayant une autre longueur d'onde (longueur d'onde d'excitation). On entend par zone non fluorescente une zone pouvant soit recevoir la longueur d'onde d'excitation mais ne pouvant pas fluorescer soit une zone capable d'absorber ou réfléchir la longueur d'onde d'excitation.

Par ailleurs, par longueur d'onde d'excitation ou de fluorescence on entend une fenêtre spectrale de longueur d'onde.

L'électrode, ou les électrodes, est, sont destinée(s) à être recouverte(s) par un dépôt électrochimique, apte à reconnaître et à capturer des molécules-cibles qui sont sensibles à une lumière ayant la longueur d'onde d'excitation et qui sont capables d'émettre une lumière à la longueur d'onde de fluorescence, lorsque ces molécules-cibles sont excitées par la lumière à la longueur d'onde d'excitation.

Autrement dit, le couple de longueurs d'onde d'excitation et de fluorescence est caractéristique de molécules-cibles pouvant être capturées par un dépôt électrochimique recouvrant la, ou les, électrode(s).

Selon l'invention, on rend donc non fluorescente, à au moins une longueur d'onde d'excitation donnée, la surface libre du substrat récepteur du réactif.

Ceci peut être fait en utilisant des matériaux non fluorescents, ou en rajoutant une couche d'un matériau opaque à la longueur d'onde soit de fluorescence soit d'excitation pour empêcher toute fluorescence parasite, ou encore en faisant subir à la couche de surface un traitement la rendant opaque.

A titre d'exemple, on peut utiliser un polyimide, un benzocyclobutène ou une résine, le polyimide, le benzocyclobutène ou la résine pouvant être colorés ou rendus opaques par des traitements appropriés.

De plus en plus en analyse, soit médicale soit de molécules nouvelles en pharmacologie, on cherche à augmenter la sensibilité de la mesure. Les fluophores employés sont de plus en plus émissifs, les méthodes de fixations de ceux-ci sur l'analyte à évaluer de plus en plus sélectifs.

Tous ces progrès ne sont intéressants que si il n'y a pas d'autres sources parasites de fluorescence. Ceci est d'autant plus vrai lorsque le microsystème intègre des fonctions logiques d'adressage par exemple qui font appel pour le substrat support à des méthodes de fabrication micro-électronique. La présente invention permet d'utiliser toutes les ressources de la micro-électronique en terme d'intégration, sans avoir à modifier les protocoles de fabrication.

L'invention concerne donc une structure de capteur comportant un substrat à la surface duquel àes capteurs, par exemple électrochimiques, sont formés, ces capteurs étant entourés par une surface libre du substrat, cette surface libre étant opaque et non fluorescente à une longueur d'onde de fluorescence lorsqu'elle est soumise à une longueur d'onde d'excitation.

Là encore, les capteurs sont aptes à reconnaître et à capturer des molécules cibles sensibles à une lumière à une longueur d'onde d'excitation et émettant à une longueur d'onde de fluorescence lorsque les molécules cibles sont excitées par la lumière à la longueur d'onde d'excitation.

La surface du substrat est rendue non fluorescente en utilisant des matériaux non fluorescents ou en rajoutant une couche d'un matériau non fluorescent, opaque à la longueur d'onde utilisée pour empêcher toute fluorescence parasite, ou encore en faisant subir à la couche de surface un traitement la rendant opaque.

Lorsque le système est plus simple et n'est constitué que de simples sites tels que des cavités formées sur un substrat, l'invention est également applicable et permet un choix beaucoup plus large de matériaux de substrat sans avoir à les choisir non fluorescents, ce qui peut être très intéressant d'un point de vue économique.

L'invention a donc également pour objet un support pour réactif, comportant un substrat dans lequel est défini au moins un site destiné à recevoir un réactif, au moins une partie de la zone autour du site étant non fluorescente à une certaine longueur d'onde dite de fluorescence lorsqu'une lumière à une longueur d'onde dite d'excitation est dirigée vers le substrat.

Les matériaux non fluorescents et opaques utilisables sont les mêmes que pour le support d'électrodes.

Là encore, des tests optiques, pour mettre en évidence la présence d'une molécule émettant une fluorescence, seront faits avec un bon rapport signal sur bruit.

### Brève description des dessins

De toute façon, les caractéristiques et avantages de l'invention apparaîtront mieux à la lumière de la description qui va suivre. Cette description porte sur les exemples de réalisation, donnés à titre explicatif et non limitatif, en se référant à des dessins annexés sur lesquels :
- la figure 1, déjà décrite, est une vue en perspective schématique d'un support d'électrodes connu,
- la figure 2, déjà décrite, est une vue en coupe schématique d'un support d'électrodes connu, associé à un système connu de lecture des électrodes,
- la figure 3 est une vue en perspective schématique d'un support d'électrodes conforme à l'invention,
- la figure 4 est une vue en coupe transversale du support de la figure 3,
- la figure 5 illustre schématiquement un mode d'adressage des électrodes d'un support conforme à l'invention,
- la figure 6 illustre schématiquement un dépôt électrochimique sur des électrodes d'un support conforme à l'invention,
- la figure 7 est une vue en coupe schématique d'un système de lecture d'un support conforme à l'invention, et
- la figure 8 est une vue de dessus d'un support d'électrodes selon l'invention.
- les figures 9A à 9C représentent des étapes de réalisation d'un support selon l'invention.
- les figures 10A à 10B représentent des étapes de réalisation d'un support selon l'invention.

### Description détaillée de modes de réalisation de l'invention

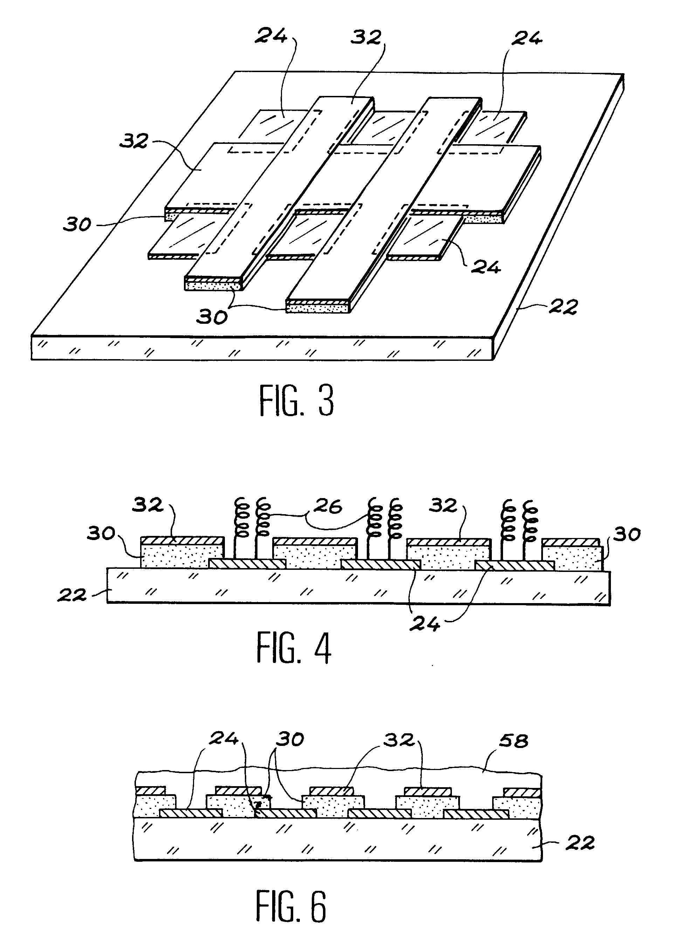

Un premier exemple de réalisation de l'invention est donné sur la figure 3, et concerne un capteur, ou support d'électrodes, comportant une matrice de capteurs électrochimiques, ou d'électrodes, 24 formé(e)s à la surface d'un substrat 20.

Chacun des capteurs ou électrodes 24 peut être recouvert par un dépôt électrochimique constitué de molécules-sondes plus simplement appelées "sondes".

Ces sondes sont aptes à reconnaître et à capturer des molécules-cibles qui sont sensibles à une lumière de longueur d'onde λ1 (longueur d'onde d'excitation) .

Parmi les capteurs, ou électrodes, 24 portant à leur surface des sondes 26 (figure 4), certain(e)s capturent des molécules-cibles tandis que les autres n'en capturent pas.

Lorsque les sondes reçoivent la lumière de longueur d'onde λ₁, celles qui ont capturé des molécules-cibles et seulement celles-là émettent une lumière de longueur d'onde λ₂ (longueur d'onde de fluorescence) qui est différente de λ₁ et que l'on peut détecter comme on le verra par la suite.

Dans l'exemple de la figure 3, le capteur, ou support d'électrodes, conforme à l'invention peut aussi comporter des moyens électroniques, non représentés sur la figure, intégrés au substrat 22, du côté de la surface de ce substrat qui porte les capteurs électrochimiques, ou électrodes 24.

Ces moyens électroniques peuvent consister en un circuit d'adressage des capteurs ou des électrodes, destiné à permettre l'étape de fonctionnalisation de ces capteurs ou électrodes, c'est-à-dire le greffage des sondes.

Ce circuit d'adressage peut aussi comporter un circuit de multiplexage suivant la complexité de la matrice de capteurs ou d'électrodes.

La structure de capteur ou le support d'électrodes conforme à l'invention comporte, en plus, des éléments électriquement isolants 30 qui sont formés sur les zones de la surface du substrat 22, zones qui sont comprises entre les électrodes 24.

Sur ces éléments isolants 30 sont respectivement formées des couches métalliques 32 qui sont opaques à la lumière de la longueur λ₁ de manière à arrêter celle-ci, ou ne donnant pas de fluorescence, en particulier à λ₂, lorsqu'un rayonnement de longueur d'onde λ₁ est dirigé vers le substrat.

Les couches métalliques 32 permettent de contrôler le potentiel électrique au voisinage des capteurs ou électrodes 24. Ce sont par exemple des contre-électrodes.

Elles forment également une obturation optique de parties non sensibles de la structure de capteur ou du support d'électrodes.

Une telle obturation optique permet d'améliorer le rapport signal/bruit lors de la lecture optique de la matrice.

La figure 4 est une vue en coupe transversale schématique de la structure de capteur ou du support d'électrode de la figure 3.

On explique ci-après une méthode d'adressage lors du dépôt électrochimique sur des capteurs ou électrodes du genre de celles qui sont représentées sur la figure 3.

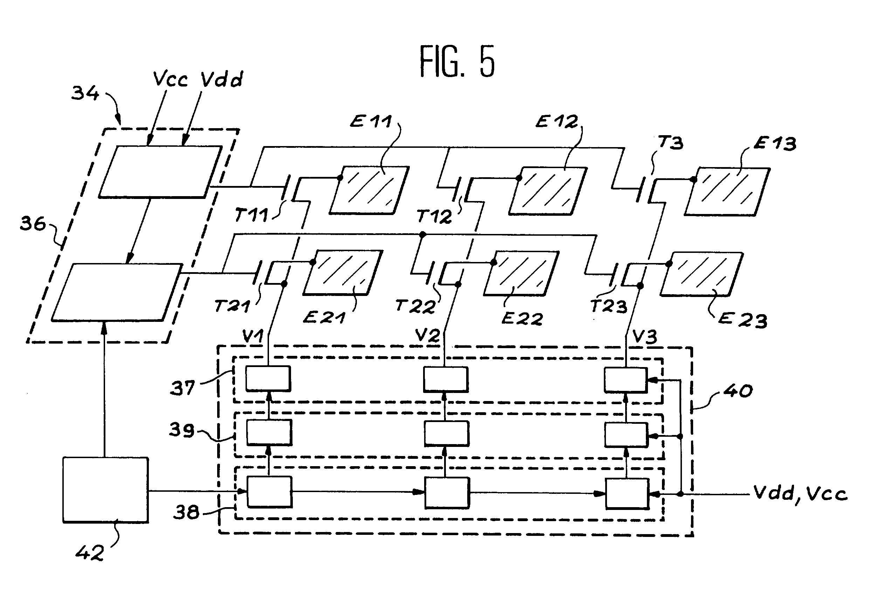

On voit sur la figure 5 une matrice de six capteurs ou électrodes, référencé(e)s E11, E12, E13 pour la première ligne de la matrice et E21, E22 et E23 pour la deuxième ligne de la matrice.

On voit également sur la figure 5 un circuit électronique 34 de commande de ces capteurs ou électrodes.

Ce circuit électronique comprend un registre à décalage 36 ainsi qu'un autre registre à décalage 38.

Ce registre 38 est associé à un registre de transfert parallèle de type « *latch »* 39 combiné à un étage d'amplification à gain 1 de type « *buffer »* 37, l'ensemble constitué par l'étage 37, le registre 38 et le registre 39 ayant la référence 40.

Chaque capteur ou électrode Eij est associé(e) à un transistor Tij, l'indice i prenant l'une quelconque des valeurs 1 et 2 et l'indice j prenant l'une quelconque des valeurs 1, 2 et 3.

Les deux registres 36 et 38 sont commandés par une horloge 42.

Le registre 36 comporte deux sorties aptes à fournir l'une un signal S1 et l'autre un signal S2 respectivement aux deux lignes de la matrice, par l'intermédiaire des transistors qui lui sont associés.

Plus précisément, les transistors Tij sont des transistors à effet de champ.

Le drain de chaque transistor est relié à l'électrode correspondante ou au capteur correspondant.

Les grilles des transistors associés à la première ligne de la matrice sont toutes reliées à la première sortie du registre 36 pour recevoir le signal S1.

Les grilles des transistors associés à la deuxième ligne de la matrice sont toutes reliées à la deuxième sortie du registre 36 pour recevoir le signal S2.

Les sources des deux transistors associés à la première colonne de la matrice de capteurs ou d'électrodes sont toutes deux reliées à une première sortie de l'étage d'amplification 37 pour qu'on puisse leur appliquer une tension contrôlée référencée V1.

Les sources des deux transistors associés à la deuxième colonne de la matrice de capteurs ou d'électrodes sont toutes deux reliées à une deuxième sortie de l'étage d'amplification 37 pour qu'on puisse leur appliquer une autre tension contrôlée référencée V2, éventuellement distincte de V1.

Les sources des deux transistors associés à la troisième colonne de la matrice de capteurs ou d'électrodes sont toutes deux reliées à une troisième sortie de l'étage d'amplification 37 pour qu'on puisse leur appliquer une autre tension contrôlée référencée V3, éventuellement distincte de V1 et de V2.

Sur la figure 5, Vcc et Vdd représentent les tensions d'alimentation des registres 36 et 38.

On peut ainsi faire l'adressage des capteurs ou des électrodes de façon séquentielle, ligne par ligne, en utilisant la structure matricielle de ces capteurs ou électrodes.

Chacune des tensions V1, V2 et V3 est susceptible de prendre des valeurs qui sont soit inférieures au seuil de tension caractéristique Vs de la fonctionnalisation sélective (capteur ou électrode non sélectionné(e)) soit supérieures à ce seuil (capteur ou électrode sélectionné(e)).

Le transfert de charge n'intervient que lorsque les signaux de sélection S1, S2 sont supérieurs au seuil des transistors, ce qui est une condition pour que les transistors soient passants (état « on »).

Par exemple, le transistor T11 est non sélectionné lorsque V1 est inférieure à la tension de seuil Vs bien que le signal S1 soit « on » et il n'y a alors pas de dépôt sur l'électrode correspondante ou le capteur correspondant.

Le transistor T11 est également non sélectionné lorsque V1 est supérieure à la tension Vs mais S1 est « off » c'est-à-dire inférieure au seuil du transistor.

Dans le cas où simultanément S1 est « on » et V1 est supérieure à Vs, le dépôt se produit sur le capteur ou l'électrode E11 associé(e) au transistor T11.

Ce mode de sélection, permet de rendre sélectivement fonctionnelles les capteurs ou électrodes formé(e)s à la surface d'un substrat tel que le substrat 22 qui, dans les exemples représentés sur les figures 3 à 5, constitue un « biochip ».

On explique ci-après la réalisation des signaux S1, S2, V1, V2 et V3.

Ces signaux sont obtenus de la façon expliquée ci-après, qui est connue pour les afficheurs.

Les signaux S1 et S2 résultent du registre à décalage 36 de la figure 5.

Les signaux de tension V1, V2 et V3 sont issus des tensions Vcc et Vdd par l'intermédiaire de l'ensemble 40, un transfert d'informations ayant lieu simultanément lors de la commutation du registre à décalage 36 et du registre de transfert parallèle 39.

Ces fonctions sont réalisables :
- soit par une électronique extérieure (connexions amovibles sur les capteurs ou électrodes)
- soit en réalisant les circuits électroniques à la surface du substrat 22 qui porte les capteurs ou électrodes (le procédé de fabrication étant alors identique à celui des transistors associés à la matrice de capteurs ou d'électrodes).

On explique ci-après les modes de dépôt.

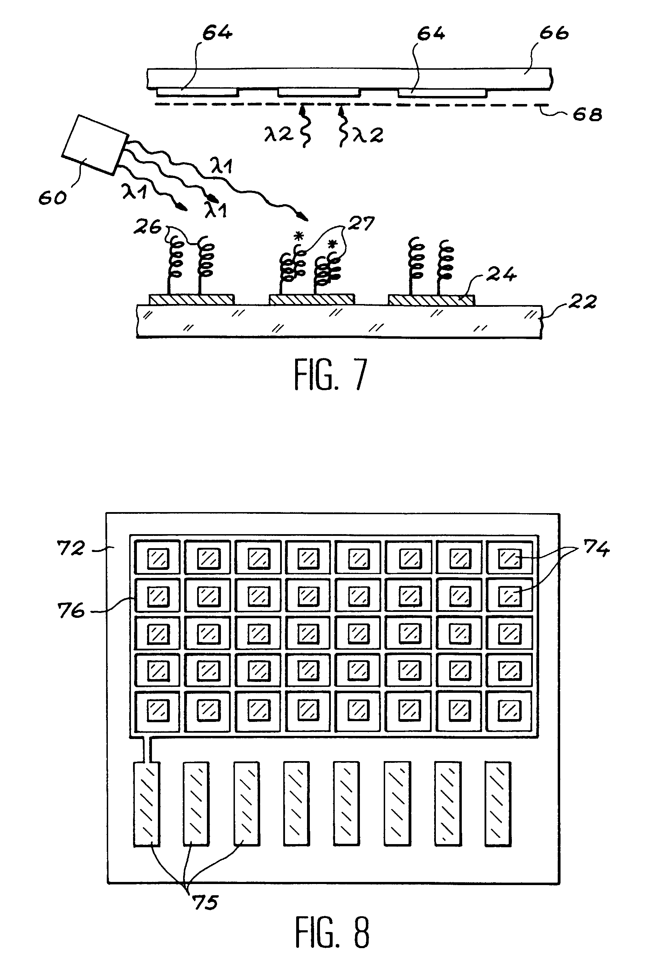

On voit sur la figure 6 la structure de capteur ou le support d'électrode comprenant le substrat 22, les capteurs ou les électrodes 24 et des contre-électrodes 32.

Ce support ou cette structure est plongé(e) dans un bain 58, par exemple un bain de polypyrrole, qui est contenu dans un récipient.

Les capteurs, ou électrodes, 24 et 32 sont relié(e)s à un potentiostat (non représenté sur la figure) pour réaliser les dépôts sélectifs sur les capteurs ou électrodes 24.

La lecture de la structure de capteur ou du support d'électrodes est expliquée en faisant référence à la figure 7 où l'on voit en coupe transversale le substrat 22 portant les capteurs ou électrodes 24 qui sont respectivement associé(e)s à des molécules-sondes 26 dont certaines portent une molécule-cible marquée 27 tandis que les autres n'en portent pas.

Le système de lecture permettant la lecture du support des capteurs ou électrodes 24 comprend une source lumineuse 60 destinée à émettre la lumière de longueur d'onde λ₁.

Cette source 60 est placée, par rapport au substrat 22, par exemple comme on le voit sur la figure 7.

Les molécules-sondes qui sont marquées ou, plus exactement, qui portent une molécule-cible marquée et qui reçoivent cette lumière de longueur d'onde λ₁ émettent à leur tour une lumière de longueur d'onde λ₂.

Le système de lecture comprend également des moyens 62 de détection de la lumière de longueur d'onde λ₂.

Ces moyens de détection 62 comprennent une matrice 64 de photodétecteurs appropriés par exemple des détecteurs à couplage de charges (CCD).

Cette matrice 64 de photodétecteurs est formée sur un substrat approprié 66.

Les moyens de détection 62 comprennent également un filtre sélectif 68 placé en regard des détecteurs à couplage de charges et donc interposé entre ces détecteurs et les capteurs ou électrodes 24 comme on le voit sur la figure 7.

Au lieu d'une matrice de détecteurs à couplage de charges (CCD) on pourrait utiliser une matrice de détection de photons de type CMOS.

Le pas de la matrice de photodétecteurs 64 peut être égal au pas de la matrice des capteurs ou électrodes 24.

En variante, le pas des photodétecteurs peut être inférieur au pas des capteurs ou électrodes (et donc au pas des groupes de molécules-sondes), ce qui permet d'éviter un positionnement précis de ces photodétecteurs par rapport aux molécules-sondes.

On réalise par exemple le filtre sélectif 68 au moyen de couches minces (par exemple au moyen de multicouches d'oxyde de silicium, titane dont les indices de réfraction sont différents les uns des autres).

Le filtre sélectif 68 laisse passer la lumière de longueur d'onde λ₂ et absorbe ou réfléchit la lumière de longueur d'onde λ₁.

L'utilisation des couches métalliques 32 opaques ou non fluorescentes à λ₁, formées sur les couches électriquement isolantes 30 permet, conformément à la présente invention, d'améliorer le rapport signal sur bruit lors de la détection.

D'autres modes de réalisation sont possibles, permettant de rendre opaque, ou non fluorescente, tout ou partie de la surface libre du substrat 22, c'est-à-dire essentiellement des parties du substrat entourant les électrodes, ou capteurs.

La figure 8 représente un autre capteur ou support d'électrodes conforme à l'invention. En surface d'un substrat 72 sont réalisées :
- des capteurs ou électrodes 74 subissant un dépôt électrochimique et adressables individuellement,
- un réseau de contre-électrodes 76.

Lorsque le nombre de capteurs ou d'électrodes devient élevé, on peut passer à un substrat actif multiplexé.

La référence 75 désigne un ensemble d'entrées/sorties d'une puce multiplexée.

La réalisation décrite ci-dessus en liaison avec les figures 3 et 4 concerne le cas particulier où, entre les capteurs ou électrodes 74, sont réalisés des dépôts isolants sur lesquels sont formées des couches métalliques opaques à une longueur d'onde λ₁.

En fait, pour éviter une fluorescence des zones non recouvertes par un dépôt réalisé sur des capteurs ou électrodes 74 (ou par le réseau de contre-électrodes 76), on peut réaliser un support d'électrodes de différentes manières.

Par exemple, comme illustré sur les figures 9A à 9C, on peut réaliser un dépôt d'un polymère opaque avant le niveau de métal assurant la réalisation de l'électrode et éventuellement de la contre-électrode (par exemple un métal bio compatible, du type Or, Pt).

Dans une première étape (figure 9A), on réalise sur un substrat 72 le dépôt, puis la gravure d'une couche 80 de polymère naturellement opaque par exemple une résine colorée ou polyimide chargé au carbone.

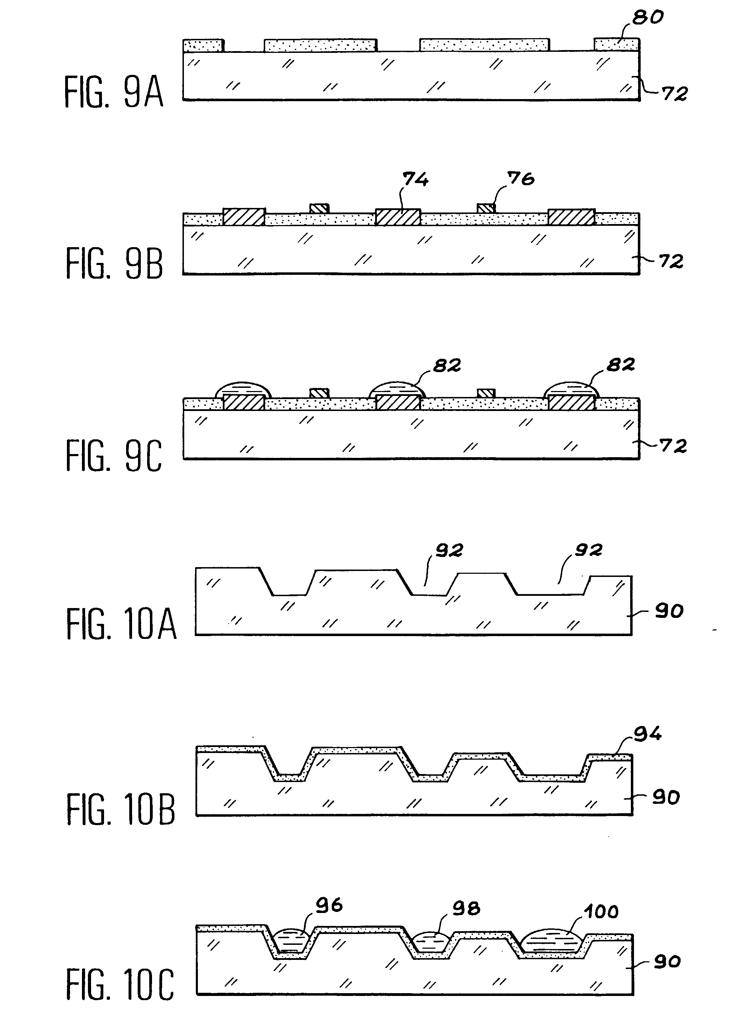

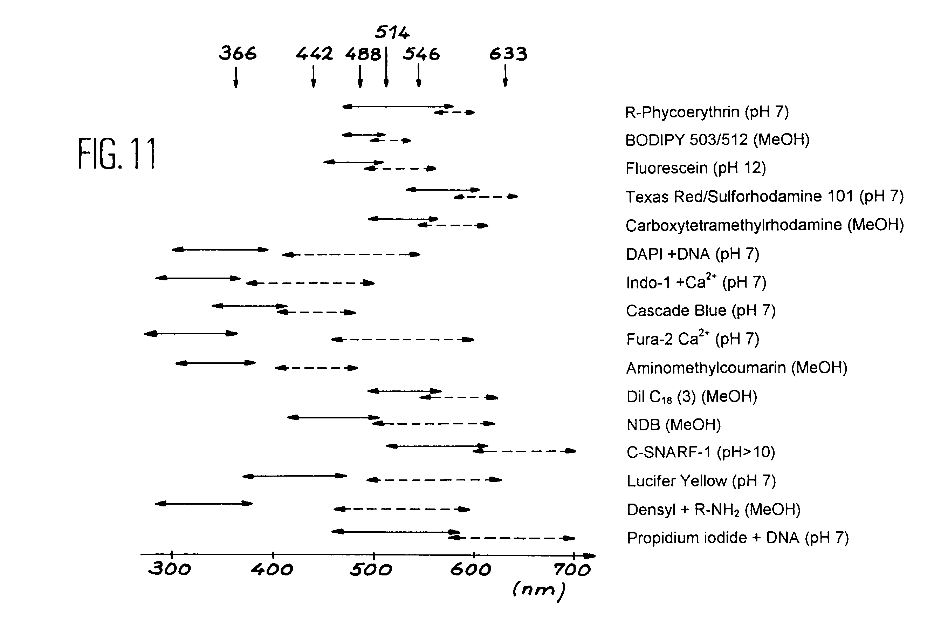

Puis (figure 9B), on dépose et on grave le métal des capteurs ou électrodes 74 et éventuellement des contre-électrodes 76.

Enfin, un dépôt électrochimique des réactifs 82 est réalisé (figure 9C).

Le polymère peut donc avantageusement être rapporté sous forme de film 80 avant d'être localement enlevé. Ceci permet d'utiliser des techniques proches des circuits-imprimés, dans lesquelles ce type de films existe.

Comme on l'observe, la couche de polymère empêche toute fluorescence du substrat lors de l'analyse ultérieure.

Selon une variante, on peut aussi utiliser un polymère non opaque qui est ensuite traité pour le devenir. C'est le cas par exemple des polyimides ou des résines photosensibles utilisées en micro-électronique. Le traitement préconisé pourra être : un recuit thermique élevé sous atmosphère contrôlée, ou un traitement chimique dénaturant en surface le polymère ou encore un traitement sous laser dénaturant en surface le polymère. L'intérêt de cette deuxième approche est d'utiliser ce qui se fait en micro-électronique, ce qui laisse la possibilité d'augmenter la densité d'intégration des capteurs ou électrodes.

Selon une autre réalisation, on dépose, au moins sur la surface du substrat en dehors des zones de capteurs ou d'électrodes 74, une couche métallique écran. Ce peut être par exemple la même couche de métal utilisée comme électrodes (Or, Pt), avec une géométrie adaptée. On peut conserver à cette couche la fonction contre-électrode, qui devient élargie et qui recouvre une grande partie du substrat. Lorsque cette fonction contre-électrode n'est pas indispensable, cette zone n'est utilisée que comme écran dans le cadre de l'invention.

Dans les deux cas, il ne subsiste qu'une faible zone non recouverte de métal, ce qui limite la fluorescence parasite du substrat.

Les différents modes de réalisations de l'invention, qui viennent d'être exposés, peuvent être utilisés comme déjà décrit ci-dessus en liaison avec les figures 5 à 7.

L'invention concerne également des substrats, utilisés comme support de réactifs, mais qui ne nécessitent pas d'adressage, qu'il soit sélectif ou pas.

Un tel substrat est illustré sur la figure 10A. Le substrat 90 présente des sites tels que des cavités 92 (par exemple gravées) dans lesquelles seront déposés les réactifs, non plus électrochimiquement, mais par simple pipetage.

On dépose ensuite (figure 10B) une couche 94, métallique ou polymère opaque sur l'ensemble du substrat, avant de déposer des réactifs 96, 98, 100 dans les cavités 92.

A titre d'exemple, on peut choisir, en tant que matériau antifluorescent pour la couche 94 :
- des matériaux isolants, pour le cas où le substrat comporte, ou non, des liaisons électriques. Ces matériaux peuvent être choisis parmi les polymères époxy, les polyimides ; on peut également choisir du BCB (Benzocyclobutène). Ils peuvent être chargés en colorants opaques aux longueurs d'onde de fluorescence, ou subir un traitement thermique postérieur les rendant opaques,
- les métaux, qui sont opaques dans le visible. Mais ils ne conviennent que pour le cas où la couche 94 n'est pas en contact avec des zones conductrices.

Là encore, le support obtenu permet de réaliser des réactions locales, sur les réactifs 96, 98, 100, la réaction pouvant ensuite être mise en évidence par un dispositif (moyens d'émission à λ₁ et moyens de détection à λ₂) du type décrit ci-dessus en liaison avec la figure 7. Le rapport signal sur bruit obtenu est amélioré du fait que les surfaces libres (non occupées par du réactif) ne peuvent fluorescer.

## Revendications

1. Support d'électrodes, ce support comprenant un substrat (22, 72) et des électrodes (24, 74) qui sont formées sur ce substrat, ce support étant tel qu'au moins une partie d'une zone entourant au moins une électrode est non fluorescente à au moins une certaine longueur d'onde, dite longueur d'onde de fluorescence, lorsque cette partie est soumise à une lumière ayant une autre longueur d'onde, dite longueur d'onde d'excitation.

2. Support d'électrodes selon la revendication 1, toute la zone entourant ladite électrode étant non fluorescente à la longueur d'onde de fluorescence.

3. Support d'électrodes selon la revendication 1, toutes les zones libres entourant les électrodes étant non fluorescentes à la longueur d'onde de fluorescence.

4. Support d'électrodes selon la revendication 1, 2 ou 3, tout ou partie de la zone entourant l'électrode, ou toutes les zones libres entourant les électrodes, étant recouverte(s) d'un matériau (80) non fluorescent à la longueur d'onde de fluorescence lorsque cette partie est soumise à une lumière ayant la longueur d'onde d'excitation et opaque à la longueur d'onde de fluorescence et/ou d'excitation.

5. Support d'électrodes selon la revendication 4, ledit matériau non fluorescent (80) étant un matériau isolant opaque.

6. Support d'électrodes selon l'une des revendications 4 à 5, le matériau (80) non fluorescent s'étendant sous au moins une des électrodes.

7. Support d'électrodes selon l'une des revendications 4 à 6, ledit matériau (80) non fluorescent comportant un polyimide ou une résine photosensible opaques.

8. Support d'électrodes selon l'une des revendications 1, 2 ou 3, tout ou partie de la zone entourant l'électrode, ou bien toutes les zones libres entourant les électrodes étant recouverte(s) d'une couche conductrice écran (32), cette couche étant électriquement isolée de la ou des électrodes.

9. Support d'électrodes selon la revendication 8, la couche métallique écran (32) étant une couche en or ou en platine.

10. Support d'électrodes selon la revendication 5, des moyens de contrôle du potentiel électrique (32) étant formés sur le matériau isolant opaque (30).

11. Support d'électrodes selon la revendication 8, caractérisé en ce que la couche conductrice écran forme des moyens de contrôle du potentiel électrique.

12. Support selon l'une quelconque des revendications précédentes, comprenant en outre un circuit d'adressage qui est intégré au substrat et destiné à commander les électrodes.

13. Support selon l'une quelconque des revendications précédentes, comprenant en outre un circuit de multiplexage qui est intégré au substrat et destiné au multiplexage de signaux adressés aux électrodes.

14. Structure de capteurs comportant un substrat (22, 72) à la surface duquel des capteurs électrochimiques (24, 74) sont formés, ces capteur étant entourés par une surface libre du substrat, cette surface libre étant, au moins en partie, non fluorescente à une certaine longueur d'onde, dite longueur d'onde de fluorescence, lorsqu'elle est soumise à une lumière ayant une autre longueur d'onde, dite longueur d'onde d'excitation, inférieure à la longueur d'onde de fluorescence.

15. Support pour réactif, comportant un substrat (90) dans lequel est défini au moins un site (92) destiné à recevoir un réactif (96, 98, 100), au moins une partie de la zone autour du site étant non fluorescente à une certaine longueur d'onde λ₂, dite longueur d'onde de fluorescence, lorsqu'une lumière à une longueur d'onde λ₁ dite longueur d'onde d'excitation, est dirigée vers le substrat.

16. Support selon la revendication 15, le site étant une cavité.

17. Support selon la revendication 15, tout ou partie de la zone autour du site étant recouverte d'une couche de matériau non fluorescent et opaque à la longueur d'onde de fluorescence et/ou d'excitation.

18. Support selon la revendication 17, le matériau non fluorescent étant un isolant ou un conducteur.

19. Structure de capteur comportant un support d'électrodes selon l'une des revendications 1 à 13 ou un support pour réactif selon l'une des revendications 15 à 18.
